# EUROPEAN PATENT SPECIFICATION

(11) **EP 2 617 364 B1**
(45) Date of publication and mention of the grant of the patent: **04.06.2014**
(21) Application number: 12000340.5
(22) Date of filing: 19.01.2012
(51) Int. Cl.: A61B 17/00, A61B 17/34

(54) **Surgical instrument for positioning a sleeve**
Chirurgisches Instrument für Positionierhülse
Instrument chirurgical pour le positionnement d'un manchon

(43) Date of publication of application: 24.07.2013
(73) Proprietor: Stryker Trauma GmbH, 24232 Schönkirchen (DE)
(72) Inventor: Hirsch, Oliver, 24159 Kiel (DE); Prien, Ole, 24145 Kiel (DE); Giersch, Helge, 24235 Laboe (DE)
(74) Representative: Röthinger, Rainer

(56) References cited:
- WO-A1-96/20021
- WO-A1-2011/163402
- WO-A2-94/06681
- WO-A2-2004/052180
- JUNG ANTRIEBSTECHNIK U.AUTOMATION GMBH: "MagSpring - Magnetfedern aus einer Hand", , 9 April 2009 (2009-04-09), XP002673418, Retrieved from the Internet: URL:http://web.archive.org/web/20090409044 441/http://www.ja2-gmbh.de/magsprin.html [retrieved on 2012-04-04]

## Description

### Technical Field

The present disclosure generally relates to surgical instruments for use in surgical procedures. In particular, a surgical instrument for positioning a sleeve is described.

### Background

Surgical instrumentation is often to some extent modularized to increase the number of alternative configurations available to the surgeon, to permit an easy cleaning and sterilization thereof, and to decrease the required storage space. Examples of such modular surgical instrumentation include instrumentation for suprapatellar surgery and similar surgical procedures, where a handle is releasably attached to a tissue protection sleeve.

In suprapatellar surgery, it is known to use a sleeve with a handle for holding tissue away from the surgical site and for providing access to a region to be treated, for example inside a patient's knee. A trocar may be put into the sleeve for insertion into a vein, artery, bone marrow or body cavity. During the surgery, the surgeon manipulates the handle to control position and movement of the sleeve.

A handle configured to releasably hold a sleeve is known from WO 2004/052180. The handle includes an opening into which the sleeve is inserted. A retractable slider pin is spring-biased to protrude in its extended position into the opening. The sleeve comprises complementary recesses around its circumference for being engaged by the slider pin at a desired angular position relative to the handle.

One problem associated with the handle of WO 2004/052180 is the fact that cleaning and sterilization procedures may become difficult. One factor contributing to this difficulty is the presence of a helical spring which acts upon the slider pin. The "natural" geometry of the helical spring causes biological remains from the surgery to risk to adhere to the spring. At the same time the spring, due to its geometry, is not easily accessible for a cleaning or sterilization medium. Even if the spring was enclosed by a sleeve, biological remains risk to enter the space of the spring through the play around the slider pin and to remain in the space of the spring.

### Summary

Accordingly, there is a need for a surgical instrument for positioning a sleeve which facilitates cleaning and sterilization procedures.

A surgical instrument for positioning a sleeve is provided, wherein the surgical instrument comprises a shaft, a handle connected to one end of the shaft, a sleeve holder connected to the opposite end of the shaft and defining a space in which the sleeve is to be positioned, and a movable locking element adapted to protrude into the space for engagement of the sleeve, wherein the instrument further comprises at least one magnet adapted to force the locking element into the space by a magnetic force.

The at least one magnet may be a permanent magnet or an electromagnet. Moreover, the surgical instrument may substantially be comprised of non-magnetic material. As an example, one or more of the handle, the shaft and the sleeve holder may consist of a non-magnetic material. The magnet may be connected to or comprised by the locking element.

The locking element be movable along the shaft or otherwise. It may be forced into the space of the sleeve holder by a repelling magnetic force. In such a realization, the magnet (and thus the locking element) may be repelled by a magnetic part at the front end of the handle and/or a magnetic part at the back end of the shaft into the space. According to one realization of this aspect, a first magnet is connected to the handle or the shaft and at least one second magnet is connected to or comprised by the locking element, and wherein the magnets face each other with a pole of the same type.

Alternatively, the locking element may be forced into the space by an attracting magnetic force. As an example, the magnet, if connected to or comprised by the locking element, may attract a magnetic part (which need not be a magnet itself but may consist of or comprise, for example, iron or any other magnetizable material) at the front end of the shaft and/or the sleeve holder and/or the sleeve itself. In such a realization, the magnet (and thus the locking element) may thus be attracted into the space. In case the magnetic part is a magnet itself, the magnets may face each other with poles of different types. Of course, the locking element itself could also consist of a magnetizable material (e.g., in a case in which the magnet is comprised by the sleeve).

The sleeve holder may be adapted to hold the sleeve around a circumference of the sleeve. The circumference of the sleeve may be circular or non-circular. A polygonal shape of the sleeve may be used. It is also possible to arrange one or more openings in the sleeve holder around its circumference.

The sleeve holder and the locking element may be adapted to hold the sleeve with at least three contact points located along more than 180° of a circumference of the sleeve. It is also possible to realize the sleeve holder as two jaws or in the form of a circular or non-circular opening. The jaws may be flexible and the sleeve may be inserted into the sleeve holder in a direction substantially perpendicular to the longitudinal axis of the sleeve.

The sleeve holder may be adapted to orient the sleeve such that a longitudinal axis of the sleeve is substantially perpendicular to a longitudinal axis of the shaft. In order to provide a more ergonomic working position for the operator, it is possible to orient the sleeve holder such that a longitudinal axis of the sleeve has an angle of less than 90° compared to the longitudinal axis of the shaft. This angle may be, for example, 70° degrees.

The locking element may be movable along a longitudinal axis of the shaft. Furthermore, the locking element may have the shape of a rod. The rod may have an elongated cylindrical form. The cross section of the rod may be of a circular or polygonal shape.

The end of the locking element, which is adapted to protrude into the space, may have a chamfered portion. The chamfered portion may be a flat surface between the lower longitudinal side and a front side of the locking element.

The locking element may be arranged inside the shaft. The locking element and the shaft may have matching (e.g., circular) cross sections.

A control portion may be connected to the locking element for retracting the locking element out from the space. The control portion may have a material and/or shape for providing a high frictional surface to the operator.

According to a first variant, the control portion may be releasably attached to the locking element. In this case, one or more points around the periphery of the locking element may be in contact with the inner periphery of the shaft. The control portion may furthermore be arranged to partially cover the shaft (e.g., to be guided along the shaft).

According to a second variant, the control portion may enclose the locking element. In this case, one or more points around the periphery of the control portion may be in contact with the inner periphery of the shaft. The control portion may have a cross section which matches the cross sections of the shaft and the locking element.

The locking element may alternatively be arranged enclosing the shaft around a circumferential direction. In this case, since the shaft needs to be connected to the sleeve holder, only a part around the circumference of the locking element may protrude into the space.

According to a further realization, the shaft may have an aperture at a circumferential portion of the shaft. The aperture may extend along the longitudinal axis of the shaft along a space defined by the back end of the movable parts inside the shaft and the front end of the handle.

For example, if a magnet is arranged at the back end of the locking element and a magnet is arranged at the front end of the handle, the aperture in the shaft may extend from the front end of the magnet on the handle to the back end of the magnet on the locking element when the locking element is in its most forward position.

If the control portion is arranged as partially covering a part of the shaft, the aperture may be located at an opposite side around the circumference of the shaft with respect to the control portion. The extension of the aperture around a circumferential direction of the shaft may be for example 180° or more as long as the strength of the shaft is not compromised.

The control portion may be connected to the locking element through an opening at the circumferential portion of the shaft. The control portion may enclose the locking element around the circumferential direction. The control portion may cover the opening both when the locking element is in its most forward position and in its most backward position. The play between the control portion and the outer circumference of the shaft may made be as small as possible without compromising the movability of the control portion relative to the shaft. In the same way, the play between the control portion and the inner circumference of the shaft may be made as small as possible without compromising the movability of the control portion relative to the shaft.

The control portion may partially enclose the shaft in circumferential direction. The control portion may be arranged with an overlap to the outer side of the shaft, both in a longitudinal and in a circumferential direction. The control portion may furthermore enclose the shaft around about the half of the circumference of the shaft.

The surgical instrument comprises the sleeve described herein. The sleeve may be realized as a tissue protection sleeve. In the case the locking element has a chamfered portion, the sleeve may be inserted into the sleeve holder along the longitudinal axis of the sleeve from a direction where the chamfered portion is located.

It is also possible that the sleeve to be positioned in the sleeve holder has a chamfered portion at its upper end which urges the locking element to a retracted position when inserting the sleeve into the sleeve holder. In this case, the operator does not need to retract the locking element when inserting the sleeve into the sleeve holder and the locking element does not need to be provided with a chamfered portion.

The sleeve may have at least one recess adapted to receive the locking element. In one implementation, the sleeve has multiple such recesses around its circumference.

In the case the locking element has a chamfered portion, the sleeve may be provided with a radially outwardly protruding portion for engagement with the sleeve holder. This arrangement prevents any movement of the sleeve relative to the sleeve holder along the longitudinal axis of the sleeve. This portion may also be provided to the sleeve even if the locking element does not have a chamfered portion.

It will be appreciated that a surgical system comprising the surgical instrument may comprise additional components. Such further components may include at least one of a drill guide (e.g., in the form of a sleeve) and a trocar for insertion into the sleeve mounted to the handle.

### Brief Description of the Drawings

Further details, advantages and aspects of the present disclosure will become apparent from the following embodiments taken in conjunction with the drawings, wherein:
- Fig. 1: shows a top view of an embodiment of a surgical instrument;
- Fig. 2: shows a cross section along line A-A of Fig. 1;
- Fig. 3: shows a close-up view of the front portion of the locking element;
- Fig. 4: shows a top view of a surgical instrument without a control portion;
- Fig. 5: shows a cross section of a surgical instrument connected to a sleeve; and
- Fig. 6: shows a cross section of a surgical instrument with an aperture at the circumferential portion of the shaft.

### Detailed Description

In the following, several embodiments of a surgical instrument will be described. The same reference numerals will be used to denote the same or similar structural features of the surgical instrument.

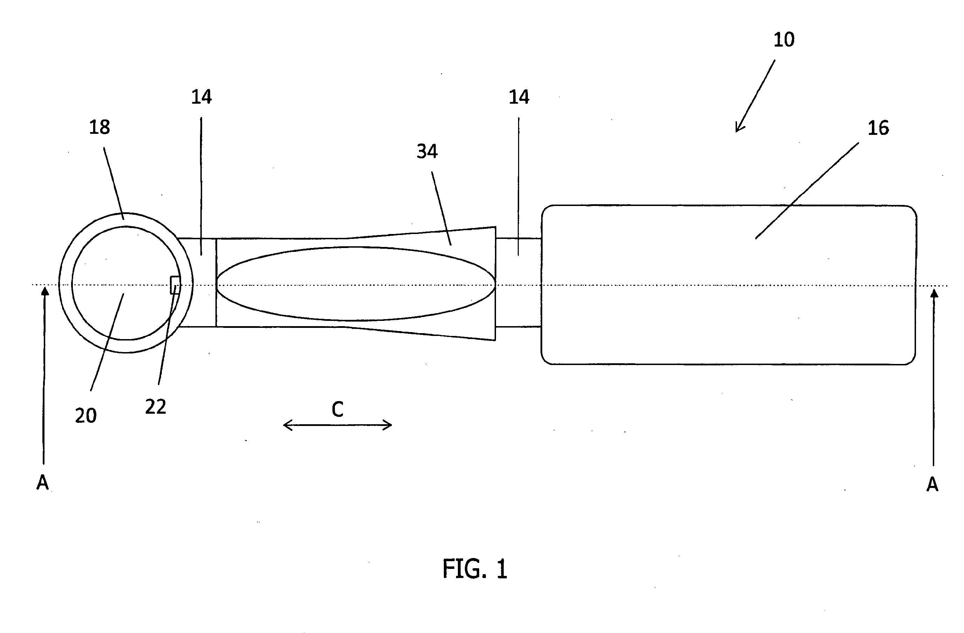

Fig. 1 shows a top view of an embodiment of a surgical instrument 10. The surgical instrument 10 comprises a shaft 14, a handle 16 connected to one end of the shaft 14 and a sleeve holder 18 connected to the opposite end of the shaft 14. The sleeve holder 18 defines a space 20 in which a sleeve is to be positioned. A locking element 22 is moveable along the shaft 14 and adapted to protrude into the space 20 for engagement of the sleeve. A control portion 34 is illustrated on top of the shaft 14 and connected to the locking element 22. The control portion 34 is here illustrated as having an oval high-friction surface for being manually engaged by a surgeon.

Throughout the description, a forward direction is referred to as a direction along arrow C in Fig. 1 towards the sleeve holder 18 and a backward direction is referred to as a direction along arrow C towards the handle 16. Correspondingly, a forward end or a front end of a part refers to an end of the part closest to the sleeve holder 18 and a back end of a part refers to an end of the part closest to the handle 16. Furthermore, an upper end or top of a part is an end at the same side as the control portion 34 and a lower end of a part is an end opposite to the upper end or top.

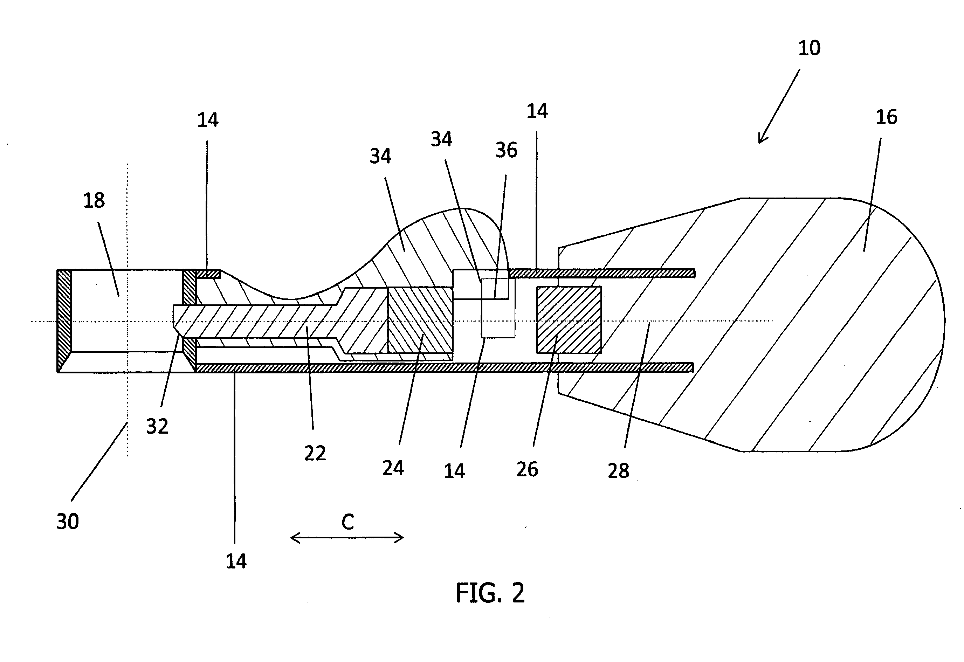

Fig. 2 illustrates a cross sectional view of the surgical instrument 10 in Fig. 1 along line A-A. As becomes apparent from Fig. 2, the sleeve holder 18 is provided with a chamfered portion along a circumference of its lower end. A longitudinal axis of the shaft 14 is denoted by 28. A longitudinal axis of the sleeve holder 18 is denoted By 30.

With reference to Figs. 1 and 2, the control portion 34 is illustrated on top of the shaft 14 and rigidly connected to the locking element 22. The control portion 34 and the locking element 22 are moveable back and forth along direction C. It may be desired that the play between the locking element 22 and the sleeve holder 18 is made as small as possible without compromising the movability of the locking element 22 relative to the sleeve holder 18. A seal ring may further be considered between the locking element 22 and the sleeve holder 18.

The locking element 22 has a chamfered portion 32 at its front end side. The angle between the chamfered portion 32 and the lower longitudinal side of the locking element 22 is approximately 45°. The edge between the chamfered portion 32 and the lower longitudinal side of the locking element 22 is positioned at an inner side of the circumference of the sleeve holder 18 when the locking element 22 is in its most forward position.

The shaft 14 has an opening 36 at its top portion. The opening 36 may have a rectangular shape. A first magnet 24 is connected to the back end of the locking element 22. The control portion 34 is enclosing the locking element 22 and the magnet 24. Furthermore, the control portion 34 protrudes through the opening 36. The control portion 34 may also enclose the back end of the magnet 24.

The control portion 34 is abutting against the front side of the opening 36 of the shaft 14 and against the sleeve holder 18. The control portion 34 covers the entire opening 36 of the shaft 14 when the locking element 22 is in its most forward position. The inside of the control portion 34 is thereby visible through the opening 36 in this cross section.

A second magnet 26 is connected to the front end of the handle 16. The two magnets 24, 26 face each other with a pole of the same type. The distance between the magnets 24, 26 is short enough for the magnets 24, 26 to interact with a repelling magnetic force. The locking element 22, the magnet 24 and the control portion 34 are thereby forced by the repelling magnet force between the magnets 24, 26 into the most forward position illustrated in Figs. 1 and 2 such that the front end of the locking element 22 protrudes into the space 20 defined by the sleeve holder 18.

The locking element 22, the magnet 24 and the control portion 34 can be retracted against the magnetic force between the magnets 24, 26. The locking element 22 may at least be able to be retracted so that the front end of the locking element 22 flushes with the inner circumference of the sleeve holder 18.

When the control portion 34 is manually pulled back, the locking element 22 exits from the space 20. To this end, the operator may wrap his hand around the handle 16 and pull the control portion 34 backward with the thumb of the same hand in order to retract the locking element 22 from the sleeve holder 18.

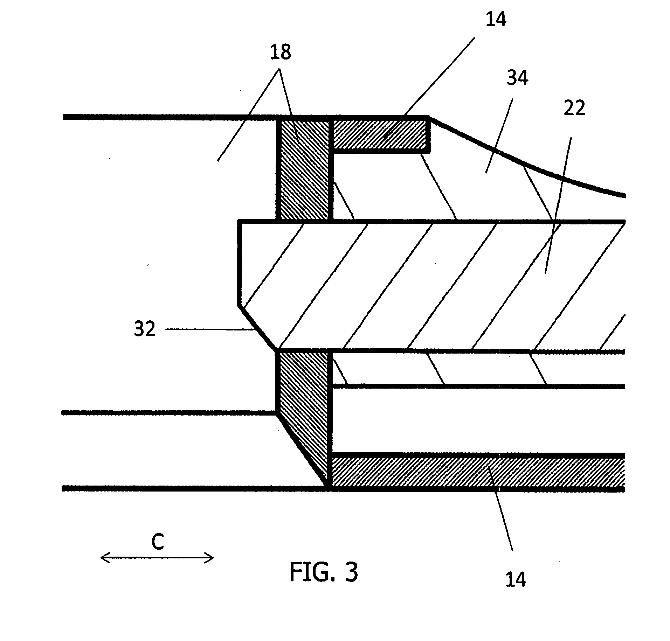

Fig. 3 illustrates a close-up view of the front end portion of the locking element 22 in the position illustrated in Fig. 2. The front upper side of the control portion 34 is here aligned with the top portion of the shaft 14. The control portion 34 may alternatively overlap the front end of the shaft 14 when the locking element 22 is in its most forward position. Alternatively, the control portion 34 may overlap the front end of the shaft 14 when the locking element 22 is in its most backward position without protruding over the inner circumference of the sleeve holder 18 when the locking element 22 is in its most forward position.

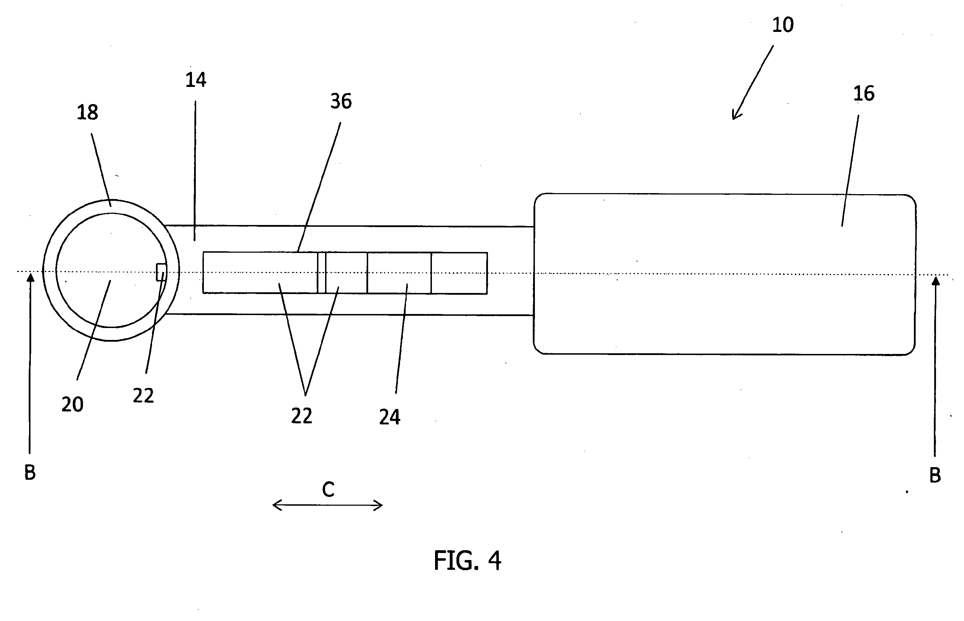

Fig. 4 illustrates a top view of a surgical instrument 10 without the control portion 34. The edges of the radially outwardly protruding part of the locking element 22 are visible through the opening 36. An operator may thereby retract the locking element 22 by contacting these edges. The magnet 24 connected to the back end of the locking element 22 is also visible through the opening 36.

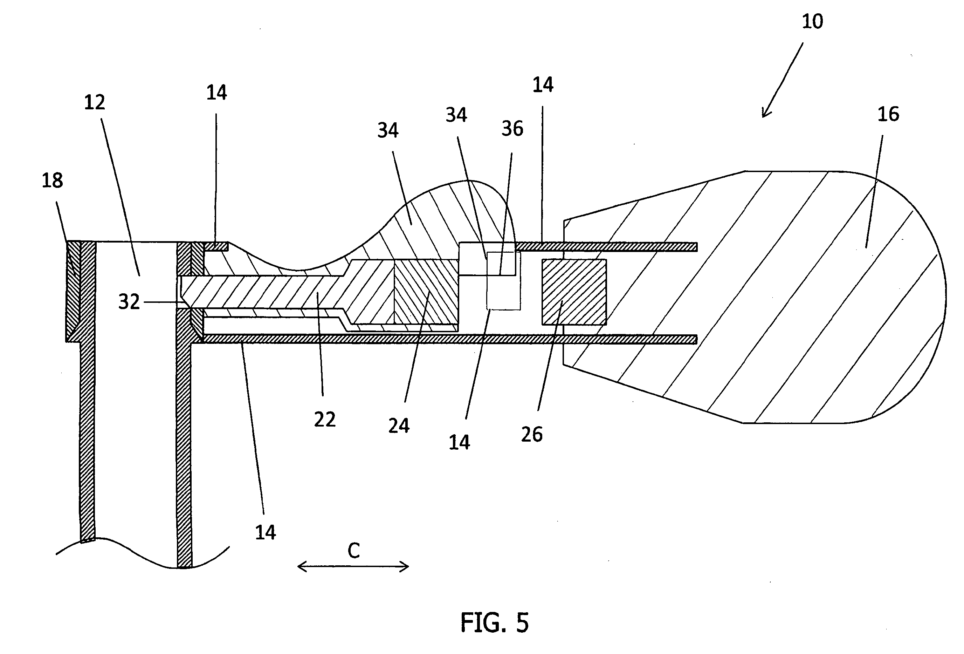

Fig. 5 shows a cross section along lines B-B in Fig. 4 but with a tissue protection sleeve 12 connected to the sleeve holder 18 (here, the control portion 34 is again connected to the locking element 22 and the magnet 24). The surgical instrument 10 is thus in an operational condition.

As illustrated in Fig. 5, the front end of the locking element 22 protrudes into a recess of the sleeve 12. The sleeve 12 is thereby rotationally locked and locked against a movement down along the longitudinal axis 30 of the sleeve holder 18 (see Fig. 2). The sleeve 12 is provided with a radially outwardly protruding portion around the circumference of its lower end. This portion engages with a lower portion of the sleeve holder 18, and the sleeve 12 is thereby locked against a movement up relative to the sleeve holder 18.

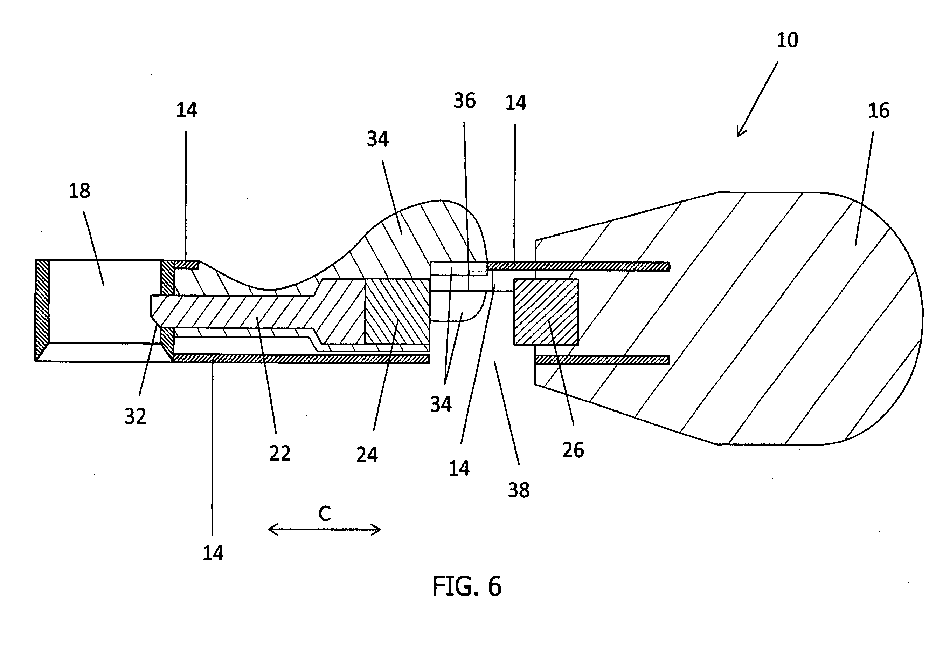

Fig. 6 shows a cross section of a surgical instrument 10 according to a further embodiment. Also in this embodiment, the locking element 22, the magnet 24 and the control portion 34 are illustrated in their most forward position.

As shown in Fig. 6, an aperture 38 is arranged along a part of the circumference of the shaft 14 in a region of a back end of the locking element 22. The aperture 38 facilitates entry of a cleaning or sterilization medium (such as hot water vapor) into the shaft 14.

The inner circumference of the back end of the control portion 34 is visible both through the opening 36 and through the aperture 38. The aperture 38 extends in a longitudinal direction from the front end of the handle 16 to the back end of the magnet 24.

The surgical instrument 10 comprising the sleeve 12 presented herein may be used in connection with a wide variety of surgical procedures including suprapatellar surgery. In suprapatellar surgery, the surgical instrument 10 may be used in connection with intramedullary nailing. In this regard, the sleeve 12 may be positioned to protect surfaces of the knee joint during, for example, drilling and nailing procedures. The sleeve 12 may be used to accommodate and guide one or both of a drill sleeve and a trocar.

After completion of the surgery, it is desired that the involved instrumentation can be disassembled, cleaned and sterilized easily. As has become apparent from the above embodiments, the provision of one or more magnets (instead of helical springs) facilitates the cleaning and sterilization procedures, and the risk for adhesion of biological remains is decreased. Specifically, the utilization of one or more magnets enables the provision of a surgical instrument with smooth surfaces to which biological remains to a lesser extent risk to adhere. The provision of an aperture in the shaft further improves the accessibility to clean and sterilize the movable parts.

While the present disclosure has been described with reference to exemplary embodiments, it will be appreciated that the present invention is not limited to what has been described above. It will, for example, be appreciated that the dimensions of the parts may be varied as needed. Accordingly, it is intended that the present invention be limited only by the scope of the claims appended hereto.

## Claims

1. A surgical instrument (10) for positioning a sleeve (12), the surgical instrument comprising: a shaft (14), a handle (16) connected to one end of the shaft (14), a sleeve holder (18) connected to the opposite end of the shaft (14) and defining a space (20) in which the sleeve (12) is to be positioned and a movable locking element (22) adapted to protrude into the space (20) for engagement of the sleeve (12),
**characterized in that** the instrument further comprises at least one magnet (26) adapted to force the locking element (22) into the space (20) by a magnetic force.

2. The surgical instrument (10) of claim 1, wherein the locking element (22) is forced into the space (20) by a repelling magnetic force.

3. The surgical instrument (10) according to claim 1 or 2, wherein a first magnet (26) is connected to the handle (16) or the shaft (14) and at least one second magnet (24) is connected to or comprised by the locking element (22), and wherein the magnets (24,26) face each other with a pole of the same type.

4. The surgical instrument (10) according to claim 1, wherein the locking element (22) is forced into the space (20) by an attracting magnetic force.

5. The surgical instrument (10) according to any of the preceding claims, wherein the sleeve holder (18) and the locking element (22) are adapted to hold the sleeve (12) with at least three contact points located along more than 180 degrees of a circumference of the sleeve (12).

6. The surgical instrument (10) according to any of the preceding claims, wherein the locking element (22) is movable along a longitudinal axis of the shaft (14).

7. The surgical instrument (10) according to any of the preceding claims, wherein the locking element (22) has the shape of a rod.

8. The surgical instrument (10) according to claim 7, wherein the end of the locking element (22), which is adapted to protrude into the space (20), has a chamfered portion (32).

9. The surgical instrument (10) according to any of the preceding claims, wherein the locking element (22) is arranged inside the shaft (14).

10. The surgical instrument (10) according to claim 9, wherein the shaft (14) has an aperture (38) at a circumferential portion of the shaft (14).

11. The surgical instrument (10) according to any of the preceding claims, comprising a control portion (34) connected to the locking element (22) for retracting the locking element (22) out from the space (20).

12. The surgical instrument (10) according to claim 11, wherein the control portion (34) is connected to the locking element (22) through an opening (36) at a circumferential portion of the shaft (14).

13. The surgical instrument (10) according to claim 11 or 12, wherein the control portion (34) partially encloses the shaft (14) in a circumferential direction.

14. The surgical instrument (10) according to any of the preceding claims, further comprising a sleeve (12).

15. The surgical instrument according to claim 14, wherein the sleeve (12) has at least one recess adapted to receive the locking element (22).

## Patentansprüche

1. Chirurgisches Instrument (10) zum Positionieren einer Hülse (12), wobei das chirurgische Instrument umfasst:
einen Schaft (14), einen Griff (16), welcher mit einem Ende des Schafts (14) verbunden ist, eine Hülsenhalterung (18), welche mit dem gegenüberliegenden Ende des Schafts (14) verbunden ist und eine Aussparung (20) definiert, in welcher die Hülse (12) zu positionieren ist, und
ein bewegliches Verriegelungselement (22), welches ausgebildet ist, in die Aussparung (20) zum Einwirken auf die Hülse (12) hineinzuragen,
**dadurch gekennzeichnet, dass** das Instrument weiter mindestens einen Magneten (26) umfasst, welcher ausgebildet ist, das Verriegelungselement (22) durch eine magnetische Kraft in die Aussparung (20) zu drängen.

2. Chirurgisches Instrument (10) nach Anspruch 1, wobei das Verriegelungselement (22) durch eine abstoßende magnetische Kraft in die Aussparung (20) gedrängt wird.

3. Chirurgisches Instrument (10) nach Anspruch 1 oder 2, wobei ein erster Magnet (26) mit dem Griff (16) oder dem Schaft (14) verbunden ist und mindestens ein zweiter Magnet (24) mit dem Verriegelungselement (22) verbunden oder davon umfasst ist, und wobei die Magneten (24, 26) einander mit einem gleichartigen Pol gegenüberliegend angeordnet sind.

4. Chirurgisches Instrument (10) nach Anspruch 1, wobei das Verriegelungselement (22) durch eine anziehende magnetische Kraft in die Aussparung (20) gedrängt wird.

5. Chirurgisches Instrument (10) nach einem der vorangehenden Ansprüche, wobei die Hülsenhalterung (18) und das Verriegelungselement (22) ausgebildet sind, die Hülse (12) an mindestens drei Kontaktpunkten zu halten, welche sich entlang mehr als 180° eines Umfangs der Hülse (12) befinden.

6. Chirurgisches Instrument (10) nach einem der vorangehenden Ansprüche, wobei das Verriegelungselement (22) beweglich entlang einer Längsachse des Schafts (14) ist.

7. Chirurgisches Instrument (10) nach einem der vorangehenden Ansprüche, wobei das Verriegelungselement (22) die Form einer Stange aufweist.

8. Chirurgisches Instrument nach Anspruch 7, wobei das Ende des Verriegelungselements (22), welches ausgebildet ist, in die Aussparung (20) hineinzuragen, einen abgeschrägten Abschnitt (32) aufweist.

9. Chirurgisches Instrument (10) nach einem der vorangehenden Ansprüche, wobei das Verriegelungselement (22) innehalb des Schafts (14) angeordnet ist.

10. Chirurgisches Instrument (10) nach Anspruch 9, wobei der Schaft (14) einen Ausschnitt (38) an einem Umfangsabschnitt des Schafts (14) aufweist.

11. Chirurgisches Instrument (10) nach einem der vorangehenden Ansprüche, welches einen Steuerabschnitt (34) umfasst, welcher mit dem Verriegelungselement (22) zum Zurückziehen des Verriegelungselements (22) aus der Aussparung (20) verbunden ist.

12. Chirurgisches Instrument (10) nach Anspruch 11, wobei der Steuerabschnitt (34) mit dem Verriegelungselement (22) durch eine Öffnung (36) an einem Umfangsabschnitt des Schafts (14) verbunden ist.

13. Chirurgisches Instrument (10) nach Anspruch 11 oder 12, wobei der Steuerabschnitt (34) den Schaft (14) teilweise in einer Umfangsrichtung umschließt.

14. Chirurgisches Instrument (10) nach einem der vorangehenden Ansprüche, welches weiter eine Hülse (12) umfasst.

15. Chirurgisches Instrument nach Anspruch 14, wobei die Hülse (12) mindestens eine Vertiefung aufweist, welche ausgebildet ist, das Verriegelungselement (22) aufzunehmen.

## Revendications

1. Instrument chirurgical (10) pour positionner un manchon (12), l'instrument chirurgical comprenant : un arbre (14), une poignée (16) connectée à une extrémité de l'arbre (14), un porte-manchon (18) connecté à l'extrémité opposée de l'arbre (14) et définissant un espace (20) dans lequel le manchon (12) doit être positionné et un élément de verrouillage (22) mobile adapté à faire saillie dans l'espace (20) pour un engagement du manchon (12),
**caractérisé en ce que** l'instrument comprend en outre au moins un aimant (26) adapté à forcer l'élément de verrouillage (22) dans l'espace (20) par une force magnétique.

2. Instrument chirurgical (10) selon la revendication 1, dans lequel l'élément de verrouillage (22) est forcé dans l'espace (20) par une force magnétique de répulsion.

3. Instrument chirurgical (10) selon la revendication 1 ou 2, dans lequel un premier aimant (26) est connecté à la poignée (16) ou à l'arbre (14) et au moins un deuxième aimant (24) est connecté à ou compris dans l'élément de verrouillage (22), et dans lequel les aimants (24, 26) se font face l'un l'autre avec un pôle du même type.

4. Instrument chirurgical (10) selon la revendication 1, dans lequel l'élément de verrouillage (22) est forcé dans l'espace (20) par une force magnétique d'attraction.

5. Instrument chirurgical (10) selon l'une quelconque des revendications précédentes, dans lequel le porte-manchon (18) et l'élément de verrouillage (22) sont adaptés à maintenir le manchon (12) avec au moins trois points de contact situés sur plus de 180 degrés d'une circonférence du manchon (12).

6. Instrument chirurgical (10) selon l'une quelconque des revendications précédentes, dans lequel l'élément de verrouillage (22) est mobile le long d'un axe longitudinal de l'arbre (14).

7. Instrument chirurgical (10) selon l'une quelconque des revendications précédentes, dans lequel l'élément de verrouillage (22) a la forme d'une tige.

8. Instrument chirurgical (10) selon la revendication 7, dans lequel l'extrémité de l'élément de verrouillage (22), qui est adaptée à faire saillie dans l'espace (20), a une partie (32) chanfreinée.

9. Instrument chirurgical (10) selon l'une quelconque des revendications précédentes, dans lequel l'élément de verrouillage (22) est agencé à l'intérieur de l'arbre (14).

10. Instrument chirurgical (10) selon la revendication 9, dans lequel l'arbre (14) a une ouverture (38) au niveau d'une partie circonférentielle de l'arbre (14).

11. Instrument chirurgical (10) selon l'une quelconque des revendications précédentes, comprenant une partie (34) de commande connectée à l'élément de verrouillage (22) pour retirer l'élément de verrouillage (22) de l'espace (20).

12. Instrument chirurgical (10) selon la revendication 11, dans lequel la partie (34) de commande est connectée à l'élément de verrouillage (22) à travers une ouverture (36) au niveau d'une partie circonférentielle de l'arbre (14).

13. Instrument chirurgical (10) selon la revendication 11 ou 12, dans lequel la partie (34) de commande renferme partiellement l'arbre (14) dans un sens circonférentiel.

14. Instrument chirurgical (10) selon l'une quelconque des revendications précédentes, comprenant en outre un manchon (12).

15. Instrument chirurgical (10) selon la revendication 14, dans lequel le manchon (12) a au moins un évidement adapté à recevoir l'élément de verrouillage (22).
